# EUROPEAN PATENT APPLICATION

(11) **EP 4 575 042 A1**
(43) Date of publication of application: **25.06.2025**
(21) Application number: 23383316.9
(22) Date of filing: 19.12.2023
(51) Int. Cl.: C25B 1/30, C02F 1/467, C25B 9/77, C25B 11/032, C25B 11/052, C25B 11/057, C25B 11/075, C25B 13/08

(54) **ELECTROLYSIS SYSTEM FOR A HOUSEHOLD APPLIANCE FOR PRODUCING HYDROGEN PEROXIDE AND HOUSEHOLD APPLIANCE**

(71) Applicant: BSH Hausgeräte GmbH, 81739 München (DE); BSH Electrodomésticos España, S.A., 50016 Zaragoza (ES)
(72) Inventor: Lazaro Villarroya, Guillermo Alberto, 50005 Zaragoza (ES); Lizan Sanchez, Julian, 50011 Zaragoza (ES); Zurro Sebastián, Alberto, 50018 Zaragoza (ES); Romero Zubeldia, Inko, 50005 Zaragoza (ES); Larrotiz Navarro, Alvaro, 50008 Zaragoza (ES)

(57) **Abstract**

The invention relates to an electrolysis system (10) for a household appliance for producing hydrogen peroxide (H₂O₂) from water and oxygen, with at least one electrolysis module (12). The module (12) comprises a water housing member (14) at least partially defining a fluid path for an aqueous electrolyte, an anode (16) which can be operatively brought into contact with the aqueous electrolyte for oxidation of H₂O, a sealing member (18), a cathode (22) which can be operatively brought into contact with air for the reduction of oxygen and the formation of H₂O₂, wherein the cathode is a Janus electrode with an hydrophilic side (40), which is operatively connectable with the aqueous electrolyte, and an hydrophobic side (42), which is operatively connectable with the air, and an air housing member (24) having at least one air passageway (26) through which the air can enter the air housing member (24) and be directed to the cathode (22). The invention further relates to a household appliance comprising such an electrolysis system (10).

## Description

The invention relates to an electrolysis system for a household appliance for producing hydrogen peroxide. The invention further relates to a household appliance comprising such an electrolysis system.

In the field of household appliances, it is often desirable to produce bleach and disinfectants in-situ for cleaning and for hygienic reasons. The main area of application is therefore in dishwashing and laundry care, but this capability may also be desirable in other household appliances such as vacuum cleaners, cleaning robots and the like.

Hydrogen peroxide (H₂O₂) has several advantages due to its antimicrobial and cleaning properties. Hydrogen peroxide can kill a wide range of microorganisms, including bacteria, viruses, and fungi. This makes it a valuable tool for sterilizing. Hydrogen peroxide breaks down into water and oxygen, making it environmentally friendly. It does not leave behind harmful residues or toxic fumes, making it a safer alternative to some chemical cleaners. Hydrogen peroxide can also be used as a color-safe bleach for laundry. It can help remove stains from clothing and brighten whites without damaging colored fabrics, as traditional chlorine bleach might. Hydrogen peroxide can also help eliminate foul odors caused by bacteria and organic matter. It can therefore be used in cleaning and disinfecting areas with persistent or pungent odors. Hydrogen peroxide can also effectively kill mold and mildew on various surfaces. It can thus be used to clean and prevent mold growth in bathrooms, kitchens, and other humid areas. In household appliances for food processing, hydrogen peroxide is also effective in killing bacteria and pathogens.

Many scientific publications in recent years have focused on developing a viable and efficient form of hydrogen peroxide electrolysis. The main limitation of the electrolytic production of hydrogen peroxide is the occurrence of adverse reactions as the concentration increases. These reactions (disproportionation, cathodic reduction, anodic oxidation) are responsible for decomposing it to form water again. For this reason, efforts are focused on finding catalysts that are selective towards the desired reaction, the most studied being transition metals and carbon-based catalysts.

Currently, the preferred electrolysis systems are two-electron reduction cells in an alkaline medium with a polymeric membrane, the reaction being catalyzed by noble metals. The main reason for the high performance of these electrolysis systems is the physical separation of the electrolytic chambers in such a way that destruction reactions at the anode are minimized. The main disadvantage of these electrolysis systems is that the membrane (PEM) is easily clogged in the presence of electrolyte ions, so that ultrapure water is required for the production of H₂O₂. Additionally, PEM membranes also imply a high cost per cell.

Other approaches include the use of chemicals such as sulphuric acid to increase electrolyte conductivity or to create a more reactive medium in which H₂O₂ is generated. However, such systems cannot be used in household appliances per se due to their complexity and the use of hazardous chemicals.

It is therefore the object of the present invention to provide an electrolysis system for producing hydrogen peroxide which can be used in household appliances, can be manufactured at low costs, and does not require hazardous chemicals. A further object of the invention is to provide a household appliance having such an electrolysis system.

These objects are achieved in accordance with the invention by an electrolysis system according to claim 1 and by a household appliance according to claim 16. Advantageous embodiments of the invention are specified in the dependent claims.

A first aspect of the invention relates to an electrolysis system for a household appliance for producing hydrogen peroxide (H₂O₂) from water and oxygen. The electrolysis system has at least one electrolysis module, wherein the module comprises a water housing member at least partially defining a fluid path for an aqueous electrolyte, an anode which can be operatively brought into contact with the aqueous electrolyte for oxidation of H₂O, and a sealing member. Further, the module comprises a cathode which can be operatively brought into contact with air for the reduction of oxygen and the formation of H₂O₂, wherein the cathode is a Janus electrode with an hydrophilic side, which is operatively connectable with the aqueous electrolyte, and an hydrophobic side, which is operatively connectable with the air, and an air housing member having at least one air passageway through which the air can enter the air housing and be directed to the cathode. In other words, it is envisaged that the electrolysis system comprises one or more modules. A module or modular assembly in the context of the present disclosure is a self-contained, standardized unit or component that is designed to produce hydrogen peroxide (H₂O₂) from water and oxygen within a household appliance. The module is designed to be easily integratable into a household appliance, making it more flexible, scalable, and efficient. Key characteristics of the module or modular assembly include standardization, which ensure that the module can be easily interchanged and integrated into various household appliances. This standardization simplifies the design and manufacturing process. A module in the sense of the present disclosure is also self-contained and with the exception of an electric power source and the needed educts (water and air) self-sufficient to perform the intended function. This modular design leads to several advantages, in particular interchangeability, scalability, ease of maintenance, and ease of integration into various types of household appliances.

The at least one electrolysis module comprises or consists of a water housing member, an anode, a sealing member, a cathode, and an air housing member, wherein the anode, the sealing member, and the cathode are preferably disposed between the water housing member and the air housing member such that the water housing member and the air housing member together form the housing of the module. The water and air housing members are at least in part separated by the sealing layer which helps with the assembly of the module and makes the module watertight. In some embodiments, the sealing layer can also define at least part of the fluid path for the electrolyte. As electrolyte, water, in particular normal tap water can be used. This means that ultrapure water is not required so that the electrolysis system can advantageously not only be used with air from the environment, but also with any domestic water connection, regular water tank or the like. No additional chemicals are required. According to the invention, the cathode is a so called "Janus" cathode which is named after Janus, a two-faced Roman god, because it has two distinct sides or faces with different properties. These two faces serve different functions in the electrochemical reactions. In the present invention, the Janus cathode has opposite wetting properties on different sides, namely adjacent hydrophilic and hydrophobic sides. For the Janus cathode, the hydrophilic side can be easily wetted by the electrolyte to provide efficient ionic transportation, while the hydrophobic side serves as an air collector and transporter, which provides for a good gas and ion transportation. This means that the electrolysis module only contains a liquid chamber to define a fluid path for the water and an active or passive airflow through the air housing member to the cathode (air-liquid cell). The general sum reaction that occurs during this process is:

2 H₂O + O₂ ---+ 2 H₂O₂

and takes place within the module in an area between the cathode and the anode. In this reaction, water (H₂O) formally reacts with oxygen (O₂) from air in the presence of electrons (e⁻) from the cathode to produce hydrogen peroxide (H₂O₂). Thus, the electrolysis system according to the invention allows in situ generation of H₂O₂ in a cost-effective and compact manner and without the need for problematic or ultrapure chemicals. Unlike conventional system comprising for instance PEM membranes or the like, the electrolysis system of the present invention is thus fully compatible with the use of tap water and contains no elements which can be obtruded by possible impurities contained in the water. The electrolysis system according to the invention can therefore be used also as retrofit solution in a wide variety of household appliances.

In an advantageous embodiment of the invention it is provided that the water housing member has a through hole through which an anode connector protrudes and/or that the air housing member has a through hole through which a cathode connector protrudes. This makes it particularly easy to make galvanic contact with the respective electrode and connect it to an external electric power source, preferably without compromising the tightness of the module housing.

In a further advantageous embodiment of the invention it is provided that the cathode has a layer system comprising a mesh current collector, a carbon black catalyst paste disposed within the mesh current collector, and an oxygen permeable gas diffusion layer, the gas diffusion layer preferably being disposed on one side of the mesh current collector. The presence of a gas diffusion layer promotes improved oxygen diffusion through the cathode. This ensures efficient oxygen supply to the electrochemical reactions, which is advantageous for the performance of the H₂O₂ production. The carbon black catalyst paste within the mesh current collector provides a high surface area for catalytic reactions, improving the utilization of the catalyst material and enhancing the overall electrochemical efficiency. The mesh current collector serves as an effective electrical conductor, efficiently collecting and distributing the generated electrical current, which results in better overall performance and reliability of the electrolysis system. This specific embodiment of the cathode also helps to distribute current and gases evenly, which contributes to an improved power output and to the durability and longevity of the cathode and the entire electrolysis system. The cathode also reduces overpotential, meaning that the electrochemical reactions proceed more efficiently with less energy loss, ultimately making the device more energy-efficient.

A particularly high hydrogen peroxide yield is in advantageous embodiments achieved in that the anode and the cathode have a distance between 0.2 mm and 5 mm, in particular between 0.4 mm and 4 mm. A small distance between anode and cathode generally improves the current distribution between them, which is directly related to the hydrogen peroxide generation yield while it also reduces the needed amperage for the electrolysis to work, resulting in a reduced energy usage compared to other devices and directly compatible with its integration into household appliances. Operational electrical parameters are in some embodiments between 2-24 V and 0.1-1.5 A.

In a further advantageous embodiment of the invention it is provided that the water housing member and the air housing member have relative recesses and relative protrusions along their edges, wherein the relative recesses of one housing member are aligned with corresponding relative protrusions of the other housing member only in a correct mounting disposition of the water housing member and the air housing member to each other. In other words, the two housing members have corresponding recesses (grooves) and protrusions (projections) that allow the housing members to be assembled only when both are correctly arranged and aligned with each other. This provides a simple way to ensure a poka-yoke or failproof mounting principle to reliably rule out incorrect assembly of the housing members.

In a further advantageous embodiment of the invention it is provided that the sealing member has notches on its edge which correspond to protrusions of the water housing member and/or of the air housing member only in a correct mounting disposition of the sealing member with regard to water housing member and/or to the air housing member.

This also achieves a poka-yoke or failproof assembly system to reliably rule out incorrect positioning of the sealing element with respect to the respective housing member(s).

In a further advantageous embodiment of the invention it is provided that the water housing member has an inlet and/or an outlet for the aqueous electrolyte. This allows for precise control over the flow of the aqueous electrolyte into and/or out of the module. This control is crucial in maintaining a consistent and optimized electrochemical process. The ability to introduce fresh electrolyte through the inlet and/or to remove electrolyte through the outlet ensures that the composition of the electrolyte remains at the desired levels. The inlet and/or outlet also facilitates the replacement and flushing of the electrolyte. This makes maintenance and cleaning of the module more straightforward, which is essential for long-term operation and preventing the buildup of impurities or byproducts. In some embodiments, the ability to adjust parameters such as the flow rate and/or the temperature of the electrolyte through the inlet and/or outlet allows for customization and optimization of the electrochemical process according to specific requirements of the associated household appliance.

In an additional beneficial embodiment of the invention, it is stipulated that the inlet and/or the outlet is formed as a through-hole. This allows the fluid path for the electrolyte to be defined in a particularly flexible and compact manner, especially in a system comprising several modules, in particular if the electrolyte is to be fed serially through the modules. Additionally and/or alternatively it is provided that the inlet and the outlet are disposed on opposite sides of the water housing member, in particular diagonally opposite each other. It is particularly preferred if the inlet is arranged at the bottom and the outlet diagonally opposite of the inlet at the top of the water housing member in the installed state of the module in the household appliance. The spatial arrangement of the inlet and outlet facilitates the removal of bubbles generated within the module during the reaction, eliminating the necessity for any additional bleeding mechanism. Additionally and/or alternatively it is provided that the air housing member comprises a conduit for conducting the aqueous electrolyte to the inlet and/or out of the outlet of the water housing member. This also represents a simple constructive solution to define the fluid path in a particularly flexible and compact way, in particular in an electrolysis system with two or more electrolysis modules.

In a further advantageous embodiment of the invention, it is provided that the water housing member and the air housing member are, preferably circumferentially, connected to each other by fastening means. This provides a robust and water and gas tight connection, as the combined forces of the two housing members are distributed across their shared connection. This also increases the overall strength and durability of the system. Alternatively or additionally it is provided that the water housing member and/or the air housing member has recessed receptacles for assembly means, in particular for screws and/or hexagon nuts. Recessed receptacles protect the assembly means from external elements, reducing the risk of corrosion and damage over time. The use of recessed receptacles also facilitates the assembly process, making it easier to install or disassemble the system's components. By protecting the assembly means from direct exposure to water, humidity, or corrosive substances, the recessed receptacles can further extend the life of the assembly means and thus of the housing of the electrolysis module.

In a further advantageous embodiment of the invention, it is provided that the anode is materially bonded, in particular glued to the water housing member and/or that the anode consists of an iridium-tantalum-tin-titanium alloy and/or that the cathode is materially bonded, in particular glued to the air housing member. In the context of the mentioned embodiment of the invention, there are several advantages to having the anode and/or cathode materially bonded, particularly through gluing. In that the anode is glued to the "water side" case, only one side facing the cathode gets in contact with the electrolyte while ensuring that the water housing member is watertight. In that the cathode is materially bonded to the "air side", no electrolyte can escape the fluid path or cell for the electrolyte through the air housing member. A securely bonded or glued anode and/or cathode can thus prevent electrolyte leakage, which is essential for maintaining the efficiency and safety of the electrolysis system. Material bonding of the anode and/or the cathode further ensures a secure and stable connection, reducing the risk of detachment or displacement of these crucial components. The use of an iridium-tantalum-tin-titanium alloy ensures a particularly high yield of H₂O₂ and shows excellent resistance to corrosion and the chemical reactions.

In a further advantageous embodiment of the invention, it is provided that a diaphragm is disposed between the anode and the cathode, wherein the diaphragm is made from a material that is chemically resistant to H₂O₂ and to the aqueous electrolyte, in particular from a plastic material selected from polystyrene (PS), polyethylene (PE), polycarbonate (PC), and polypropylene (PP). The diaphragm acts as an interior spacer or separator and can be made of plastic with chemical resistance to hydrogen peroxide and the electrolyte (such as regular tap water) to ensure that it does not degrade during the cell's lifetime. The diaphragm's chemical resistance against the employed chemical compounds contributes to its longevity, reducing the need for frequent replacements and maintenance. The diaphragm acts as a barrier between the anode and cathode, preventing direct contact between them, which can help avoid unwanted reactions or cross-contamination between the two components. By separating the anode and cathode, the diaphragm also enhances the efficiency of electrochemical processes by maintaining the integrity of the individual components and their respective reactions. The use of the named plastic materials allows for customization based on specific application requirements, ensuring compatibility with different chemicals, temperatures, and operational conditions.

In certain embodiments, further advantages result from the fact that the sealing member consists of an elastic deformable polymer with chemical resistance to H₂O₂ and to the aqueous electrolyte, in particular silicone. The sealing member or layer is in other words made of a flexible polymer with chemical resistance to hydrogen peroxide and to the electrolyte to ensure that the sealing member does not degrade during the system's lifetime while allowing the sealing member to be reversibly compressed in order to give the housing the desired water tightness. Silicone and similar elastic deformable polymers exhibit strong chemical resistance to substances like hydrogen peroxide and aqueous electrolytes, ensuring the sealing member's durability and integrity. The sealing member's elastic and deformable nature helps create a tight and reliable seal, preventing the leakage of H₂O₂ or electrolytes. The deformable nature of the polymer allows it to maintain its sealing capabilities even in conditions involving temperature variations or mechanical stress.

In a further advantageous embodiment of the invention, it is provided that the water housing member and the air housing member are formed as a one-piece part. In other words, the water housing member and the air housing member are formed in one piece or as a single piece, that is, as an integral component. The water housing member can be realized on one side and the air housing member on the other side of this one-piece housing construction element. This makes it particularly easy to realize several modules by stacking the individual one-piece housing construction elements with one side performing the task of the water housing and the other side performing the task of the air housing.

In further embodiments, it has been shown to be advantageous if the electrolysis system comprises two or more electrolysis modules, which are arranged and/or connected in series and/or in parallel. Using multiple electrolysis modules allows for a higher production capacity of the desired hydrogen peroxide. The ability to arrange the modules in series or parallel makes the system scalable, allowing for adjustment to meet varying production needs. Having multiple modules also provides redundancy, ensuring continuous operation even if one module fails or requires maintenance. Arranging modules in series can improve the overall efficiency of the electrolysis process, as the output of one module can serve as the input for the next. Parallel and series configurations provide flexibility in how the system can be adapted to different operational requirements and conditions. The ability to adjust the number and arrangement of modules allows for better control over the system's operation and output. Using multiple smaller modules may in some embodiments be more cost-effective than using a single, large module, especially when redundancy and scalability are required.

In a further advantageous embodiment of the invention, it is provided that the modules are configured such that the aqueous electrolyte can pass serially through all modules, in particular along a zig-zag-path, and/or wherein all anodes are connected to a common metal contact and/or wherein all cathodes are connected to a common metal contact. A serial passage of the aqueous electrolyte through all modules along a zig-zag path ensures efficient exposure to the electrolysis process, enhancing the overall reaction rate. A common path for the electrolyte stream further simplifies the management of the aqueous electrolyte, reducing the need for complex distribution systems. Connecting all anodes to a common metal contact and/or all cathodes to a common metal contact simplifies the electrical connections, thereby reducing wiring complexity. The common metal contact configuration also improves the electrical efficiency of the system, minimizing voltage losses associated with wiring and connections. Such simplified electrical connections make maintenance and servicing of the electrolysis modules more straightforward and accessible. This configuration also reduces the space requirements of the electrolysis system, making it more space-efficient, which can be advantageous in various household appliances.

A second aspect of the invention relates to a household appliance, comprising at least one electrolysis system according the first aspect of the invention. The household appliance according to the invention thus provides an electrolysis system for producing hydrogen peroxide which can be used in household appliances of different types, can be manufactured at low costs, and does not require hazardous chemicals. Further features and advantages thereof are apparent from the descriptions of the first aspect of the invention, wherein advantageous embodiments of the first aspect of the invention are to be regarded as advantageous embodiments of the second aspect of the invention and vice versa. The household appliance is preferably an appliance that comes into contact with food, water, or other materials that can harbor bacteria or germs and may need to be disinfected. The household appliance can also have bleaching properties to maintain hygiene or to treat laundry. Non-limiting examples are dishwasher, washing machines, drying machines, refrigerators, microwaves, blender and food processors, cooking appliances, coffee maker, toaster, humidifiers, air dryers, and garbage disposal appliances.

Additional features of the invention can be derived from the claims, the figures, and the figure description. The features and combinations of features mentioned in the description above, as well as those mentioned and/or shown solely in the figures, can be used not only in the specified combinations but also in other combinations without departing from the scope of the invention. Therefore, embodiments of the invention are to be considered as included and disclosed, which are not explicitly shown and explained in the figures but can be deduced and created from separate combinations of features from the explained embodiments. Additionally, embodiments and combinations of features are to be considered as disclosed, even if they do not encompass all the features of an originally formulated independent claim. Furthermore, embodiments and combinations of features, especially as outlined in the above explanations, are to be considered as disclosed, which go beyond or deviate from feature combinations presented in the claims. There shows:
- Fig. 1: a perspective view of an electrolysis system according to a first embodiment of the invention for a household appliance;
- Fig. 2: an exploded view of the electrolysis system according to Fig. 1;
- Fig. 3: a schematic longitudinal section of the electrolysis system according to Fig. 1;
- Fig. 4: a schematic representation of the reactions taking place during electrolysis;
- Fig. 5: a schematic perspective view of an inner side of a water housing member;
- Fig. 6: a schematic perspective view of an exterior of the water housing member;
- Fig. 7: a schematic perspective view of an exterior of an air housing member;
- Fig. 8: an exploded view of the layers of a Janus cathode;
- Fig. 9: a schematic longitudinal section of the Janus cathode;
- Fig. 10: a top view of a section of the Janus cathode;
- Fig. 11: a perspective view of the partially rolled up Janus cathode;
- Fig. 12: an exploded view of the water housing member, a sealing member, and the air housing member in an assembly arrangement;
- Fig. 13: a perspective view of the assembled water housing member, sealing member, and air housing member according to a poka-yoke connection principle;
- Fig. 14: a perspective view of the unassembled water housing member and air housing member due to an incorrect orientation with respect to each other;
- Fig. 15: a perspective view of the electrolysis system according to another embodiment of the invention with several stacked modules;
- Fig. 16: a partially exploded view of the electrolysis system according to Fig. 15;
- Fig. 17: an exploded view of a single electrolysis module of the electrolysis system according to Fig. 15;
- Fig. 18: a perspective and diagonal cut view a housing member with a fluid path for an electrolyte;
- Fig. 19: a perspective view of a one-piece air/water housing member with a cathode where the flow path of the air is drawn in symbolically;
- Fig. 20: a perspective view of two electrolysis modules where the fluid path of the electrolyte is drawn in symbolically;
- Fig. 21: a perspective, diagonal longitudinal section of the electrolysis system with the fluid path drawn in symbolically;
- Fig. 22: a perspective view of the electrolysis system, where only the anodes of the electrolysis modules are shown;
- Fig. 23: a perspective view of the electrolysis system, where only the cathodes of the electrolysis modules are shown; and
- Fig. 24: a perspective view of the electrolysis system, where only the anodes and the cathodes of the electrolysis modules are shown.

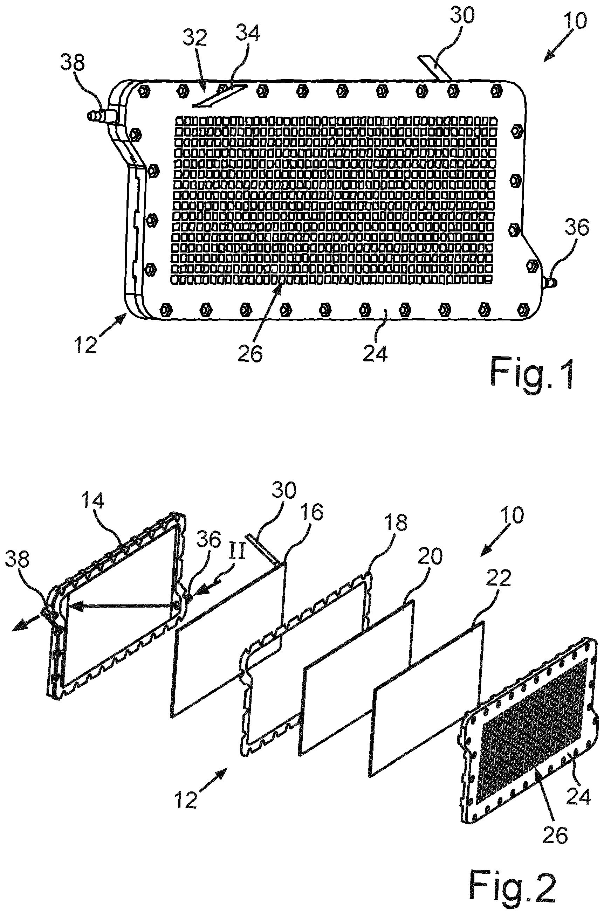

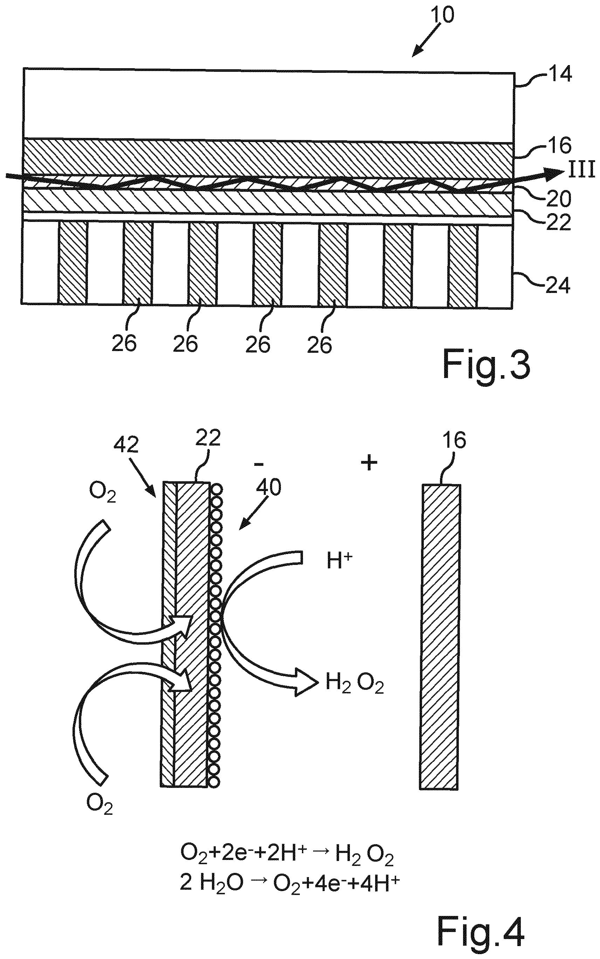

Fig. 1 shows a perspective view of an electrolysis system 10 according to an embodiment of the invention. The electrolysis system 10 will be explained in conjunction with Fig. 2, which shows an exploded view of the electrolysis system 10 according to Fig. 1, and with Fig. 3, which shows a schematic longitudinal section of the electrolysis system 10 according to Fig. 1. The electrolysis system 10 is configured for installation and use in a household appliance, for example in a laundry care appliance. The electrolysis system 10 produces hydrogen peroxide (H₂O₂) from regular tap water as an aqueous electrolyte and oxygen from the air. For this purpose, the electrolysis system 10 of the current embodiment comprises a single electrolysis module 12. The electrolysis module 12 comprises a water housing member 14 which at least partially defines a fluid path, which is marked with arrows II, for the aqueous electrolyte. The electrolysis module 12 further comprises an anode 16 which can be operatively brought into contact with the aqueous electrolyte for oxidation of H₂O, a sealing member 18 to make the module 12 watertight, an optional diaphragm 20, a cathode 22 which can be operatively brought into contact with air for the reduction of oxygen and the formation of H₂O₂, and an air housing member 24 having lattice-shaped access openings as air passageways 26 through which the air can enter the air housing member 24 and be directed to the cathode 22. The water housing member 14 and the air housing member 24 together form a housing or case of the module 12 in which the other components are enclosed. The sealing member 18 can be made from silicone.

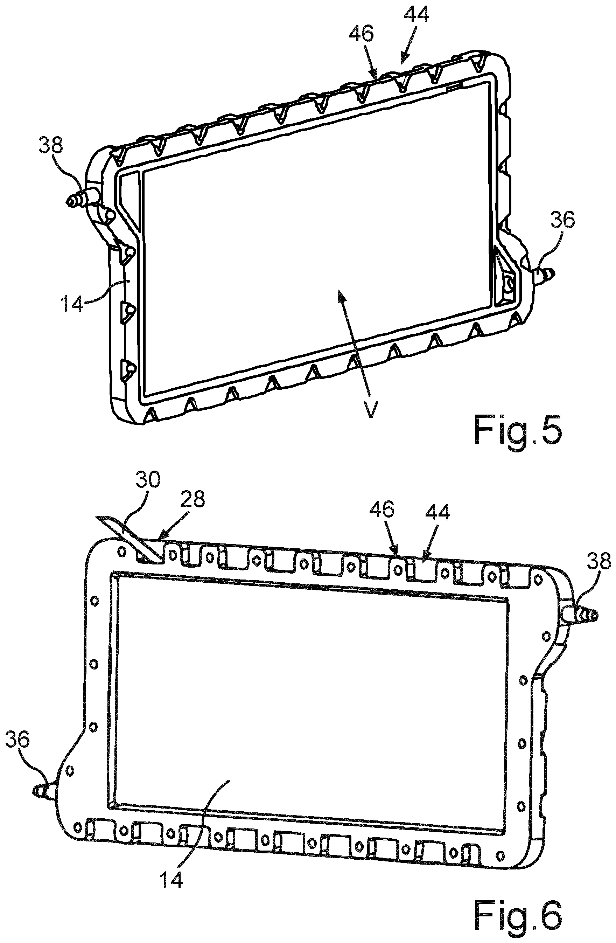

The water housing member 14 has a through hole 28 (see Fig. 6) through which an anode connector 30 protrudes. The air housing member 24 similarly has a through hole 32 through which a cathode connector 34 protrudes. The anode 16 and the cathode 22 have a distance between 0.2 mm and 5 mm, in particular between 0.4 mm and 4 mm. A reduction of the distance between the anode 16 and the cathode 22 generally improves the current distribution between them, which is directly related to the hydrogen peroxide generation yield while it also reduces the needed amperage for the module 12 to work, resulting in a reduced electrical intensity usage compared to other devices and directly compatible with the integration of the electrolysis system 10 into different household appliances. Typical operational electrical parameters are between 2-24 V and 0.1-1.5 A. The distance between the anode 16 and the cathode 22 in the present example is defined by the non-conductive diaphragm 20 which can for example be formed as a thin plastic net. The diaphragm 20 thus prevents the anode 16 and the cathode 22 from contacting each other and ensures a constant distance between those components. As can be seen in Fig. 3, the diaphragm 20 also allows for a flow of the electrolyte between the anode 16 and the cathode 22 (arrow III). The diaphragm 20 can be made of a plastic material with chemical resistance to hydrogen peroxide and to the electrolyte (e. g. regular tap water) to ensure it does not degrade during the module's lifetime. The plastic material can be selected from Polystyrene (PS). Polyethylene (PE), Polycarbonate (PC), Polypropylene (PP), or other suitable materials.

The water housing member 14 has an inlet 36 and an outlet 38 for the aqueous electrolyte. As can be seen in Fig. 1, which shows the preferred installation position of the electrolysis system 10 in the respective household appliance, the inlet 36 and the outlet 38 are placed diagonally opposite one from the other, the inlet 36 being placed in the lower area and the outlet 38 in the upper area of the electrolysis system 10. This geometrical distribution of the inlet 36 and the outlet 38 helps to remove oxygen and other gas bubbles generated within the module 12 during the generation of H₂O₂ to exit the reaction chamber naturally without the need of an additional aid of any type.

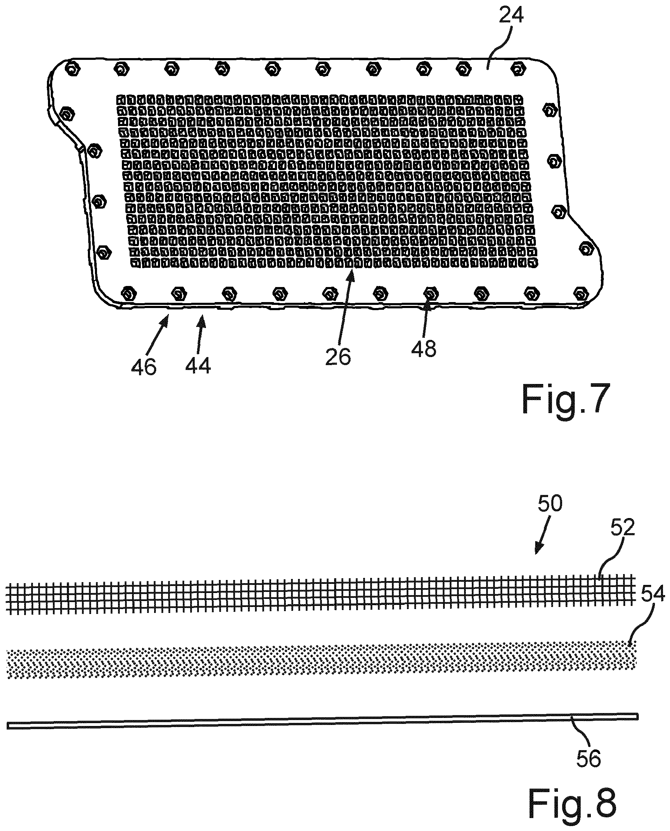

Fig. 4 shows a schematic representation of the reactions taking place during electrolysis. The cathode 22, which is shown in more detail in Fig. 8 to Fig. 11, is a so called Janus electrode with an hydrophilic side 40, which is operatively connected with the aqueous electrolyte, and an hydrophobic side 42, which is operatively connected with the air entering the air housing member 24. The anode 16 consists of a suitable material, for example of an iridium-tantalum-tin-titanium alloy. Thus, the reactions

O₂ + 2 e- + 2 H⁺ -> H₂O₂

2 H₂O -> O₂ + 4 e⁻ + 4 H⁺

formally take place during the operation of the electrolysis system 10.

Fig. 5 shows a schematic perspective view of an inner side of the water housing member 14. The inlet 36 and the outlet 38 can be seen, as well as a recessed area V used for the flow of the electrolyte.

Fig. 6 shows ca schematic perspective view of an exterior of the water housing member 14. The through hole 28 and the connector 30, which is carried out of the through hole 28, are visible. As can also be seen in Figs. 5 and 6, the water housing member 14 has relative recesses 44 and protrusions 46 along its circumference, i. e. along its edge. The relative recesses 44 can be aligned with corresponding relative protrusions 46 of the air housing member 24 (see Fig. 7) only in a correct mounting disposition of the water housing member 14 and the air housing member 24 with respect to each other. Both housing members 14, 24 can thus only be assembled in a correct relative position due to this poka-yoke principle that limits the orientation of all pieces to a required relative position and thereby ensures that all the elements are always placed in exactly the same, correct way.

Fig. 7 shows a schematic perspective view of an exterior of the air housing member 24. It is well visible that the air housing member circumferentially along its edge has several recessed receptacles 48 for assembly means, in particular for hexagon nuts, in order to assemble and connect the air housing member 24 and the water housing member 14 by corresponding fastening means, for example screws.

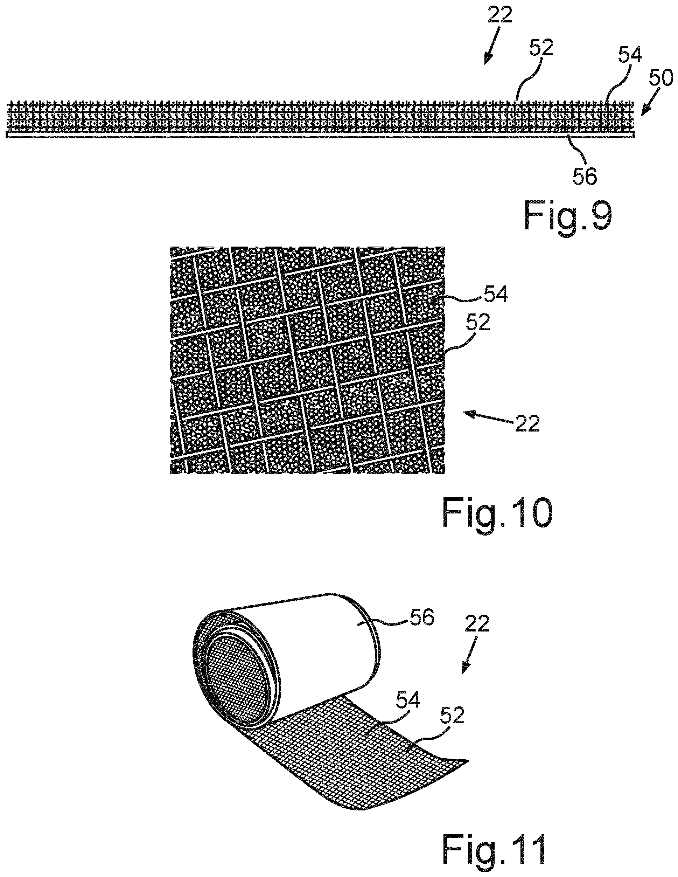

Fig. 8 shows an exploded view of the layers of the Janus cathode 22. The cathode 22 has a layer system 50 comprising a mesh current collector 52, a hydrophilic carbon black catalyst paste 54 disposed within the mesh current collector, and an oxygen permeable gas diffusion layer 56, the gas diffusion layer 56 being disposed on one side of the mesh current collector 52. In the present example, the gas diffusion layer 56, which forms the "air side" of the cathode 22, is an oxygen permeable PTFE layer. The mesh current collector 52 and the carbon black catalyst paste 54 form the "water side" of the cathode 22. Fig. 9 shows a schematic longitudinal section of the assembled Janus cathode 22 with said layer system 50 to illustrate the general structure. Fig. 10 shows a top view of a section of the Janus cathode 22. One recognizes the mesh current collector 52 and the carbon black catalyst paste 54.

Fig. 11 shows a perspective view of the partially rolled up Janus cathode 22. The cathode 22 can thus be stored as a roll and cut to the desired length in each individual case as needed for the specific electrolysis module 12.

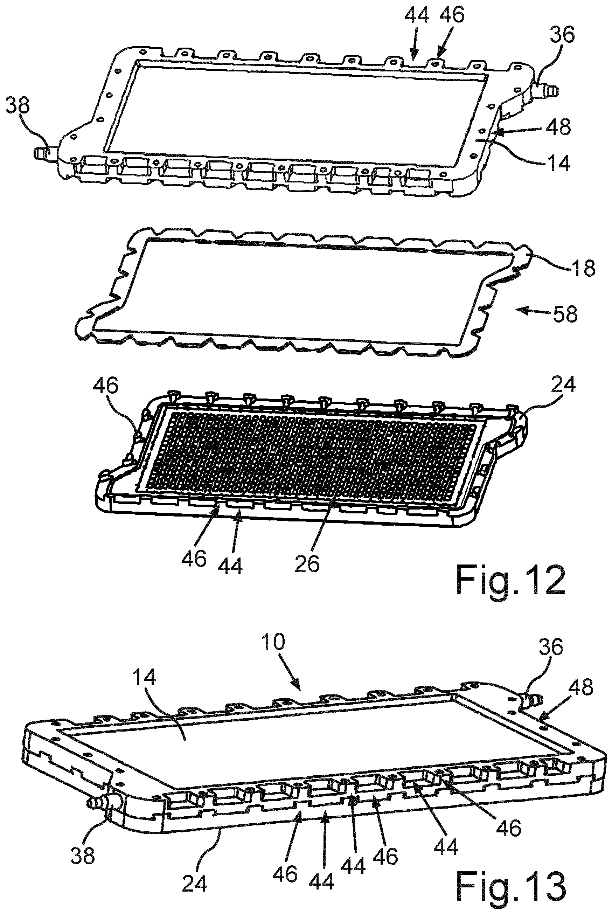

Fig. 12 shows an exploded view of the water housing member 14, the sealing member 18, and the air housing member 24 in an assembly arrangement. As can be gathered from Fig. 12, the sealing member 18 has notches 58 on its edge which correspond to protrusions 46 of the air housing member 24 only in a correct mounting disposition of the sealing member 18 with regard to the air housing member 24. This also represents a poka-yoke principle for ensuring correct relative alignment for assembly. As an alternative or in addition to the air housing member 24, the notches 58 can also correspond to protrusions 46 of the water housing member 14.

Fig. 13 shows a perspective view of the assembled water housing member 14, sealing member 18, and air housing member 24 using said poka-yoke connection principle. One can see that all components are correctly aligned with each other and are flush and thus water-tight against each other. The water housing member 14 also has relative recesses 44 and protrusions 46 on its upper surface that can also be used as a poka-yoke feature for stacking multiple modules 12 and/or for proper mounting of the electrolysis system 10 in an associated household appliance.

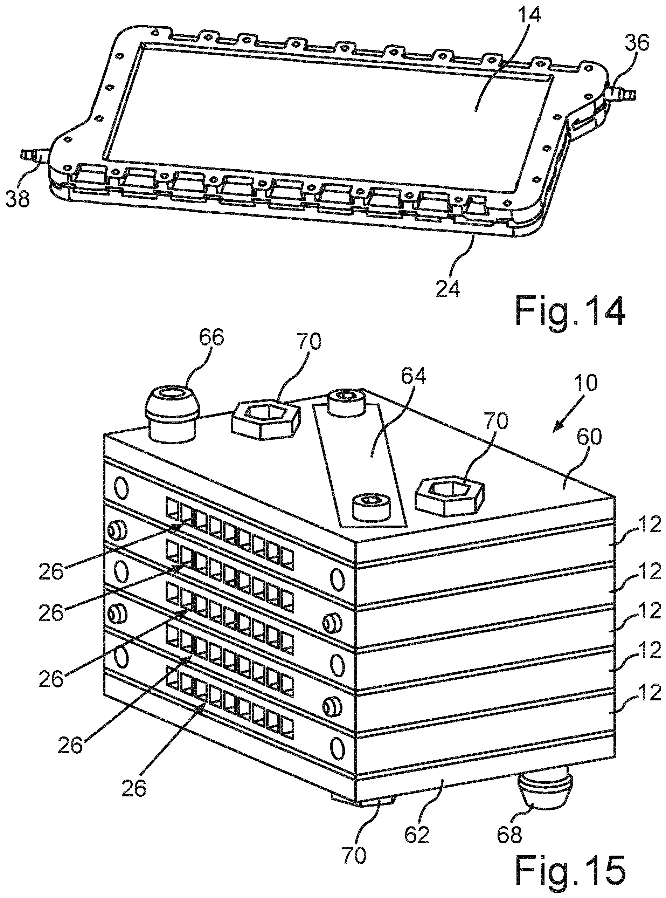

Fig. 14 shows a perspective view of the unassembled water housing member 14 and air housing member 24 due to an incorrect orientation with respect to each other. In this wrong relative orientation to each other a mounting is impossible due to the poka-yoke principle.

Fig. 15 shows a perspective view of the electrolysis system 10 according to another embodiment of the invention with several stacked modules 12. The electrolysis system 10 has an optional top plate 60 and an optional bottom plate 62, which hold several modules 12 together via screw connections 64. The number of modules 12 shown is purely exemplary and non-limiting. The top plate 60 comprises an outlet 66 for the aqueous electrolyte while the bottom plate 62 comprises an inlet 68 for the aqueous electrolyte. The top plate 60 and/or the bottom plate 62 may have additional mounting aids 70 for attachment in a household appliance.

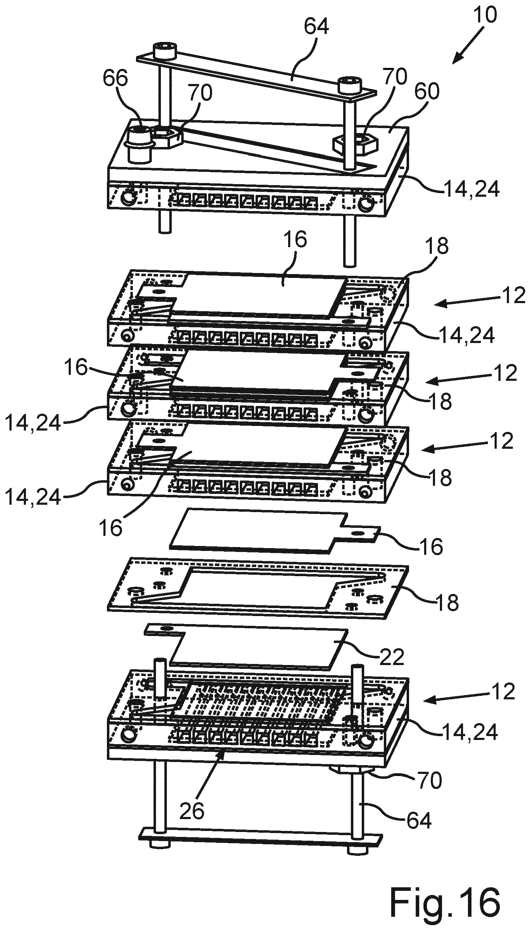

Fig. 16 shows a partially exploded view of the electrolysis system 10 according to Fig. 15. In contrast to the first embodiment, the water housing member 14 and the air housing member 24 are formed in one piece so that an upper side functions as the water housing and the lower side functions as the air housing. Furthermore, the modules 12 do not comprise a diaphragm 20. Another difference is that the sealing member 18 essentially defines the fluid path for the electrolyte.

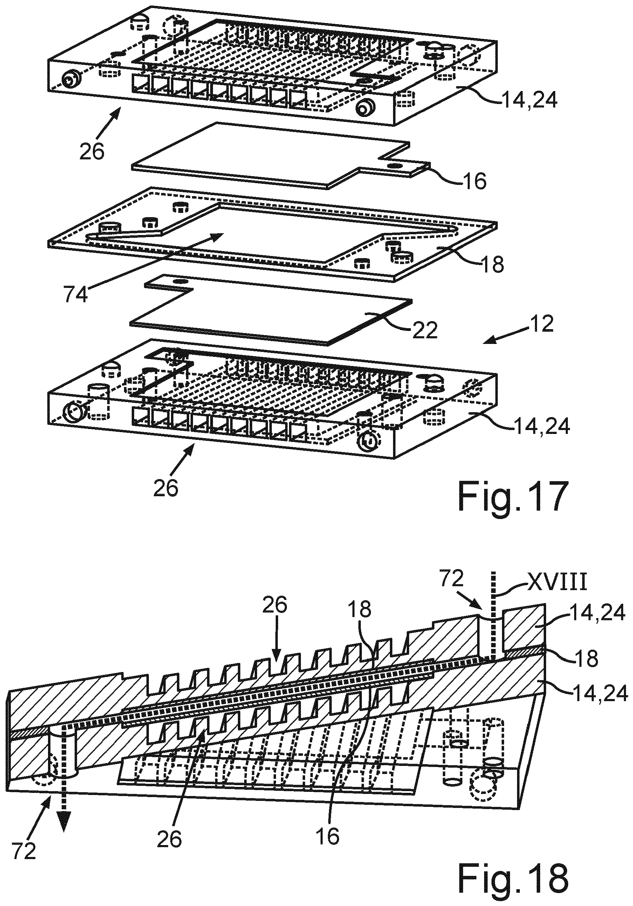

For clarification, Fig. 17 shows an exploded view of a single electrolysis module 12 of the electrolysis system 10 according to Fig. 15, while Fig. 18 shows a perspective and diagonal cut view of the assembled housing members 14, 24 with the fluid path for the electrolyte symbolized by arrow XVIII. The minimum working module 12 of this embodiment would have the upper one-piece water/air housing member 14, 24, the anode 16, the sealing member 18, the cathode and the lower one-piece water/air housing member 14, 24. The anode 16 is glued into a receptacle of the upper one-piece water/air housing member 14, 24 while the cathode is glued into a receptacle of the lower one-piece water/air housing member 14, 24. The air passageways 26 extend substantially horizontally with front and rear entry openings into the one-piece water/air housing member 14, 24. As can be seen in Fig. 18, each one-piece water/air housing member 14, 24 has conduits 72 through which the electrolyte can be fed in and out of each module 12. The distance between the anode 16 and the cathode 22 is essentially defined by the thickness of the sealing member 18 in the assembled state. As can be seen in Fig. 17, the sealing member 18 has an inner recess 74 whose geometry is matched to the position of the conduits 72.

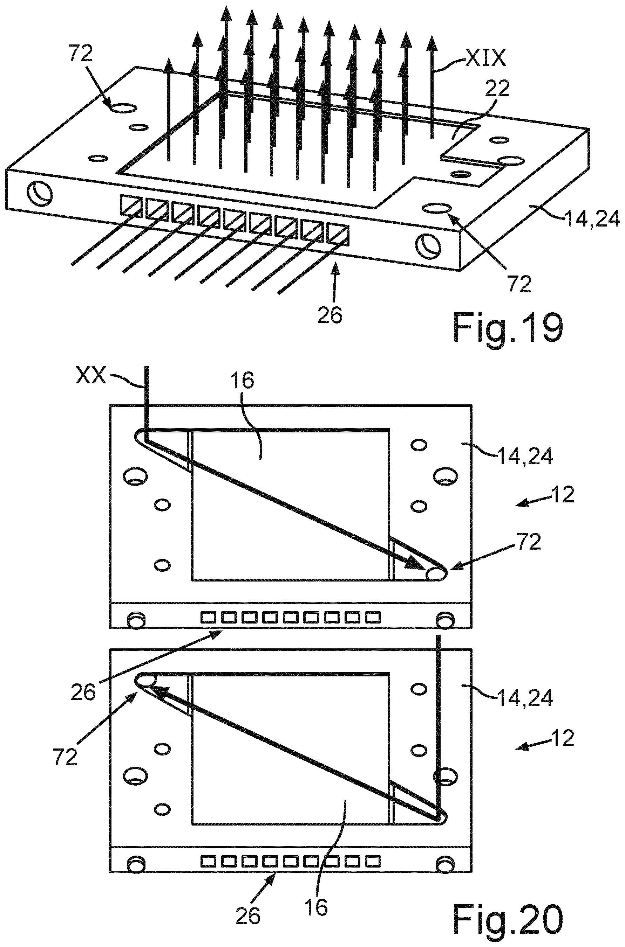

Fig. 19 shows a perspective view of the one-piece air/water housing member 14, 24 with a cathode 22 where the flow path of the air is drawn in symbolically by arrows XIX. In contrast to the first embodiment, the air does not enter from below but horizontally through the air passageways 26 and is then redirected to the cathode 22 above.

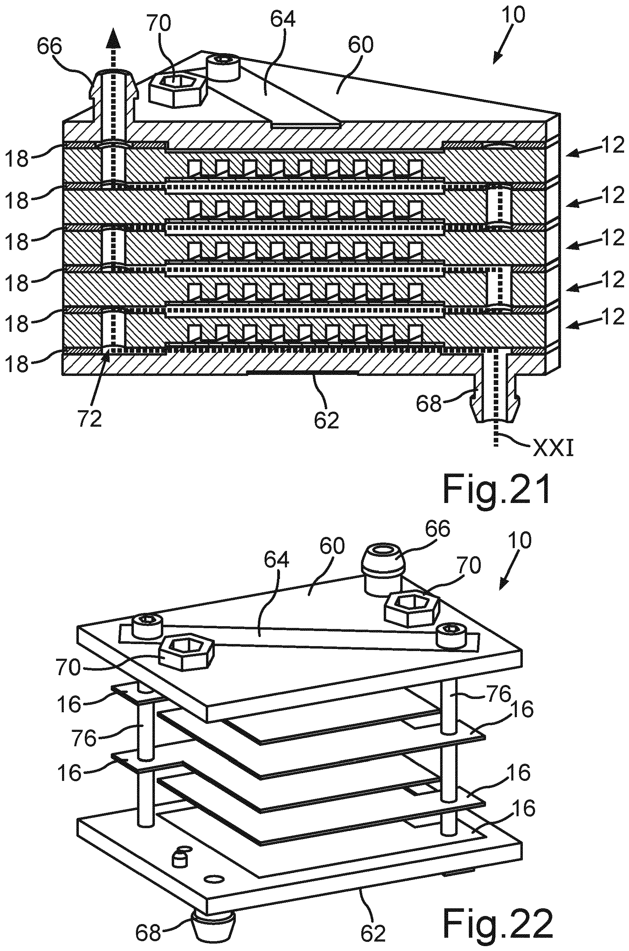

Fig. 20 shows a perspective view of two electrolysis modules 12 where the fluid path of the electrolyte is drawn in symbolically by arrow XX. As can be seen, the electrolyte is fed through the stacked modules 12 in a zigzag and serial manner. For this purpose, the modules 12 are alternately rotated by 180 degrees accordingly. In practice in the household appliance, it is preferred that the electrolyte be directed through the modules 12 from the bottom to the top to better remove air and gas bubbles from the electrolysis system 10. This is demonstrated in Fig. 21, which shows a perspective, diagonal longitudinal section of the electrolysis system 10 with the fluid path again drawn in symbolically by arrow XXI. The electrolyte can be seen flowing through the individual modules 12 and being conveyed via the conduits 72 from one module 12 to the next module 12.

Fig. 22 shows a perspective view of the electrolysis system 10, where only the anodes 16 of the electrolysis modules 12 are shown. The anodes 16 are arranged alternately, each rotated by 180 degrees which corresponds to the orientation of the individual modules 12, with the bottom anode 16, which is mounted on the bottom plate 62 being the exception.

Further, all anodes 16 are connected to a common galvanic metal contact 76 which in the present case is realized by the screw connection 64.

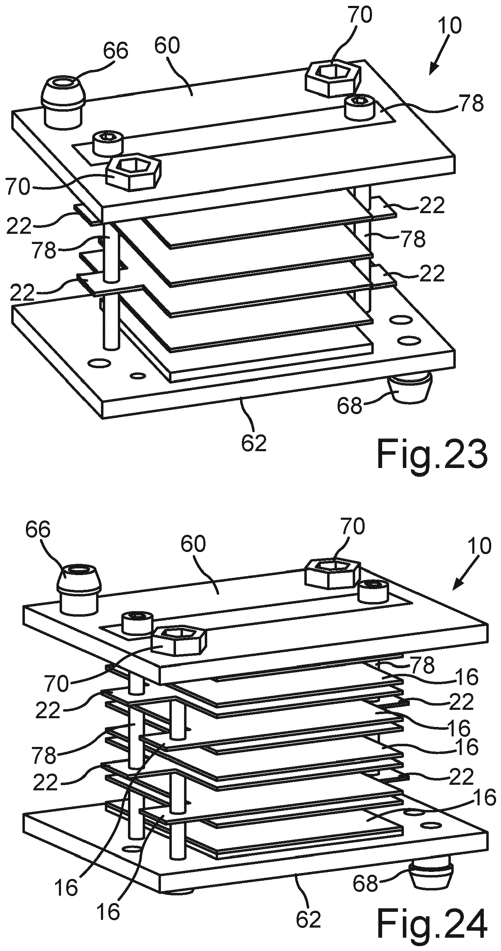

Fig. 23 shows a perspective view of the electrolysis system 10, where only the cathodes 22 of the electrolysis modules 12 are shown. Similar to the anodes 16, the cathodes 22 are alternately rotated by 180 degrees and are connected to a common metal contact 78 which is different from the metal contact 76 of the anodes 16.

Fig. 24 shows a perspective view of the electrolysis system 10, where only the anodes 14 and the cathodes 22 of the electrolysis modules 12 are shown. Here, too, the alternating orientation and electrical contacting of the anodes 16 and cathodes 22 become clear.

Alternatively, or in addition to the serial arrangement of modules 12 shown, a parallel arrangement of modules 12 or multiple electrolysis systems 10 may be provided.

The electrolysis system 10 of the invention is fully compatible with the use of tap water as it contains no elements which can be obtruded by possible impurities contained in it. The electrolysis system 10 of the invention grants the production of hydrogen peroxide without the need for expensive materials, high electrical currents, or the use of extra chemicals or consumables. The electrolysis system 10 of the invention has a shape factor that allows its use within different standard household appliances. The modular design allows an easy adaption of the hydrogen peroxide generation required for each use case. The electrolysis system 10 of the invention can be produced using standardized and generally available components with low costs per module 12.

The values for parameters outlined in the documents, which are meant to establish process and measurement conditions for assessing particular features of the invention, should be regarded as part of the invention's scope even when deviations occur. These deviations might arise from factors such as measurement inaccuracies, system malfunctions, weighing discrepancies, DIN tolerances, and similar issues.

**Reference signs**

| | | | |
|---|---|---|---|
| 10 | electrolysis system | 50 | layer system |
| 12 | electrolysis module | 52 | mesh current collector |
| 14 | water housing member | 54 | carbon black paste |
| 16 | anode | 56 | gas diffusion layer |
| 18 | sealing member | 58 | notch |
| 20 | diaphragm | 60 | top plate |
| 22 | cathode | 62 | bottom plate |
| 24 | air housing member | 64 | screw connection |
| 26 | air passageway | 66 | outlet |
| 28 | through hole | 68 | inlet |
| 30 | anode connector | 70 | mounting aid |
| 32 | through hole | 72 | conduit |
| 34 | cathode connector | 74 | inner recess |
| 36 | inlet | 76 | common metal contact anode |
| 38 | outlet | 78 | common metal contact cathode |
| 40 | hydrophilic side | | |
| 42 | hydrophobic side | | |
| 44 | recess | | |
| 46 | protrusion | | |
| 48 | receptacle | | |

## Claims

1. An electrolysis system (10) for a household appliance for producing hydrogen peroxide (H₂O₂) from water and oxygen, with at least one electrolysis module (12) comprising:
- a water housing member (14) at least partially defining a fluid path for an aqueous electrolyte;
- an anode (16) which can be operatively brought into contact with the aqueous electrolyte for oxidation of H₂O;
- a sealing member (18);
- a cathode (22) which can be operatively brought into contact with air for the reduction of oxygen and the formation of H₂O₂, wherein the cathode is a Janus electrode with an hydrophilic side (40), which is operatively connectable with the aqueous electrolyte, and an hydrophobic side (42), which is operatively connectable with the air; and
- an air housing member (24) having at least one air passageway (26) through which the air can enter the air housing member (24) and be directed to the cathode (22).

2. The electrolysis system (10) according to claim 1, wherein the water housing member (14) has a through hole (28) through which an anode connector (30) protrudes and/or wherein the air housing member (24) has a through hole (32) through which a cathode connector (34) protrudes.

3. The electrolysis system (10) according to claim 1 or 2, wherein the cathode (22) has a layer system (50) comprising a mesh current collector (52), a carbon black catalyst paste (54) disposed within the mesh current collector (52), and an oxygen permeable gas diffusion layer (54), the gas diffusion layer (54) preferably being disposed on one side of the mesh current collector (52).

4. The electrolysis system (10) according to any one of claims 1 to 3, wherein the anode (16) and the cathode (22) have a distance between 0.2 mm and 5 mm, in particular between 0.4 mm and 4 mm.

5. The electrolysis system (10) according to any one of claims 1 to 4, wherein the water housing member (14) and the air housing member (24) have relative recesses (44) and relative protrusions (46) along their edges, wherein the relative recesses (44) of one housing member (14, 24) are aligned with corresponding relative protrusions (46) of the other housing member (14, 24) only in a correct mounting disposition of the water housing member (14) and the air housing member (24) to each other.

6. The electrolysis system (10) according to any one of claims 1 to 5, wherein the sealing member (18) has notches (58) on its edge which correspond to protrusions (46) of the water housing member (14) and/or of the air housing member (24) only in a correct mounting disposition of the sealing member (18) with regard to the water housing member (14) and/or to the air housing member (24).

7. The electrolysis system (10) according to any one of claims 1 to 6, wherein the water housing member (14) has an inlet (36) and/or an outlet (38) for the aqueous electrolyte.

8. The electrolysis system (10) according to claim 7, wherein the inlet (36) and/or the outlet (38) is formed as a through-hole and/or wherein the inlet (36) and the outlet (38) are disposed on opposite sides of the water housing member (14), in particular diagonally opposite each other, and/or wherein the air housing member (24) comprises a conduit (72) for conducting the aqueous electrolyte to the inlet (36) and/or out of the outlet (38) of the water housing member (14).

9. The electrolysis system (10) according to any one of claims 1 to 8, wherein the water housing member (14) and the air housing member (24) are, preferably circumferentially, connected to each other by fastening means and/or wherein the water housing member (14) and/or the air housing member (24) has recessed receptacles (48) for assembly means, in particular for screws and/or hexagon nuts.

10. The electrolysis system (10) according to any one of claims 1 to 9, wherein the anode (16) is materially bonded, in particular glued to the water housing member (14) and/or wherein the anode (16) consists of an iridium-tantalum-tin-titanium alloy and/or wherein the cathode (22) is materially bonded, in particular glued to the air housing member (24).

11. The electrolysis system (10) according to any one of claims 1 to 10, wherein a diaphragm (20) is disposed between the anode (16) and the cathode (22), wherein the diaphragm (20) is made from a material that is chemically resistant to H₂O₂ and to the aqueous electrolyte, in particular from a plastic material selected from polystyrene (PS), polyethylene (PE), polycarbonate (PC), and polypropylene (PP).

12. The electrolysis system (10) according to any one of claims 1 to 11, wherein the sealing member (18) consists of an elastic deformable polymer with chemical resistance to H₂O₂ and to the aqueous electrolyte, in particular silicone.

13. The electrolysis system (10) according to any one of claims 1 to 12, wherein the water housing member (14) and the air housing member (24) are formed as a one-piece part.

14. The electrolysis system (10) according to any one of claims 1 to 13, comprising two or more electrolysis modules (12), which are arranged and/or connected in series and/or in parallel.

15. The electrolysis system (10) according to claim 14, wherein the modules (12) are configured such that the aqueous electrolyte can pass serially through all modules (12), in particular along a zig-zag-path, and/or wherein all anodes (16) are connected to a common metal contact (76) and/or wherein all cathodes (22) are connected to a common metal contact (78).

16. A household appliance, comprising at least one electrolysis system (10) according to any one of claims 1 to 15.

## Amended claims

### Amended claims in accordance with Rule 137(2) EPC.

1. An electrolysis system (10) for a household appliance for producing hydrogen peroxide (H₂O₂) from water and oxygen, with at least one electrolysis module (12) comprising:
- a water housing member (14) at least partially defining a fluid path for an aqueous electrolyte which is water;
- an anode (16) which can be operatively brought into contact with the aqueous electrolyte for oxidation of H₂O;
- a sealing member (18);
- a cathode (22) which can be operatively brought into contact with air for the reduction of oxygen and the formation of H₂O₂, wherein the cathode is a Janus electrode with an hydrophilic side (40), which is operatively connectable with the aqueous electrolyte and wetted by the aqueous electrolyte during operation of the electrolysis module (12), and an hydrophobic side (42), which is operatively connectable with the air and not wetted by the aqueous electrolyte during operation of the electrolysis module (12); and
- an air housing member (24) having at least one air passageway (26) through which the air can enter the air housing member (24) and be directed to the hydrophobic side (42) of the cathode (22),
- wherein the electrolysis module only provides one liquid chamber to define the fluid path for the water and an active or passive airflow through the air housing member to the cathode.

2. The electrolysis system (10) according to claim 1, wherein the water housing member (14) has a through hole (28) through which an anode connector (30) protrudes and/or wherein the air housing member (24) has a through hole (32) through which a cathode connector (34) protrudes.

3. The electrolysis system (10) according to claim 1 or 2, wherein the cathode (22) has a layer system (50) comprising a mesh current collector (52), a carbon black catalyst paste (54) disposed within the mesh current collector (52), and an oxygen permeable gas diffusion layer (56), the gas diffusion layer (56) preferably being disposed on one side of the mesh current collector (52).

4. The electrolysis system (10) according to any one of claims 1 to 3, wherein the anode (16) and the cathode (22) have a distance between 0.2 mm and 5 mm, in particular between 0.4 mm and 4 mm.

5. The electrolysis system (10) according to any one of claims 1 to 4, wherein the water housing member (14) and the air housing member (24) have relative recesses (44) and relative protrusions (46) along their edges, wherein the relative recesses (44) of one housing member (14, 24) are aligned with corresponding relative protrusions (46) of the other housing member (14, 24) only in a correct mounting disposition of the water housing member (14) and the air housing member (24) to each other.

6. The electrolysis system (10) according to any one of claims 1 to 5, wherein the sealing member (18) has notches (58) on its edge which correspond to protrusions (46) of the water housing member (14) and/or of the air housing member (24) only in a correct mounting disposition of the sealing member (18) with regard to the water housing member (14) and/or to the air housing member (24).

7. The electrolysis system (10) according to any one of claims 1 to 6, wherein the water housing member (14) has an inlet (36) and/or an outlet (38) for the aqueous electrolyte.

8. The electrolysis system (10) according to claim 7, wherein the inlet (36) and/or the outlet (38) is formed as a through-hole and/or wherein the inlet (36) and the outlet (38) are disposed on opposite sides of the water housing member (14), in particular diagonally opposite each other, and/or wherein the air housing member (24) comprises a conduit (72) for conducting the aqueous electrolyte to the inlet (36) and/or out of the outlet (38) of the water housing member (14).

9. The electrolysis system (10) according to any one of claims 1 to 8, wherein the water housing member (14) and the air housing member (24) are, preferably circumferentially, connected to each other by fastening means and/or wherein the water housing member (14) and/or the air housing member (24) has recessed receptacles (48) for assembly means, in particular for screws and/or hexagon nuts.

10. The electrolysis system (10) according to any one of claims 1 to 9, wherein the anode (16) is materially bonded, in particular glued to the water housing member (14) and/or wherein the anode (16) consists of an iridium-tantalum-tin-titanium alloy and/or wherein the cathode (22) is materially bonded, in particular glued to the air housing member (24).

11. The electrolysis system (10) according to any one of claims 1 to 10, wherein a diaphragm (20) is disposed between the anode (16) and the cathode (22), wherein the diaphragm (20) is made from a material that is chemically resistant to H₂O₂ and to the aqueous electrolyte, in particular from a plastic material selected from polystyrene (PS), polyethylene (PE), polycarbonate (PC), and polypropylene (PP).

12. The electrolysis system (10) according to any one of claims 1 to 11, wherein the sealing member (18) consists of an elastic deformable polymer with chemical resistance to H₂O₂ and to the aqueous electrolyte, in particular silicone.

13. The electrolysis system (10) according to any one of claims 1 to 12, wherein the water housing member (14) and the air housing member (24) are formed as a one-piece part.

14. The electrolysis system (10) according to any one of claims 1 to 13, comprising two or more electrolysis modules (12), which are arranged and/or connected in series and/or in parallel.

15. The electrolysis system (10) according to claim 14, wherein the modules (12) are configured such that the aqueous electrolyte can pass serially through all modules (12), in particular along a zig-zag-path, and/or wherein all anodes (16) are connected to a common metal contact (76) and/or wherein all cathodes (22) are connected to a common metal contact (78).

16. A household appliance, comprising at least one electrolysis system (10) according to any one of claims 1 to 15.
